# EUROPÄISCHE PATENTSCHRIFT

(11) **EP 1 614 405 B1**
(45) Veröffentlichungstag und Bekanntmachung des Hinweises auf die Patenterteilung: **27.11.2013**
(21) Anmeldenummer: 05001282.2
(22) Anmeldetag: 22.01.2005
(51) Int. Cl.: A61F 5/01, A61F 13/06

(54) **Medizinische Kniebandage**
Knee bandage
Genouillère

(30) Priorität: 02.07.2004 DE 202004010779 U
(43) Veröffentlichungstag der Anmeldung: 11.01.2006
(73) Patentinhaber: Bort GmbH, 71384 Weinstadt-Benzach (DE)
(72) Erfinder: Bort, Rudi, 71384 Weinstadt (DE)
(74) Vertreter: Thum, Bernhard

(56) Entgegenhaltungen:
- DE-U1- 20 207 156
- GB-A- 2 077 565
- US-A- 1 258 052
- US-A- 1 775 714
- US-A- 2 012 755
- US-A- 2 246 100
- US-A- 2 280 025
- US-A- 5 653 244

## Beschreibung

Die Erfindung bezieht sich auf eine medizinische Kniebandage mit einem Hüllkörper aus zumindest abschnittsweise elastischem Material, der an gegenüberliegenden Stirnseiten offen ist und in angelegtem Funktionszustand einen geschlossenen und um einen menschlichen Kniebereich tangential elastisch gespannten Hüllmantel bildet.

Derartige orthopädische Kniebandagen werden insbesondere verwendet, um das Kniegelenk zu stützen, zu führen und zu stabilisieren, beispielsweise bei besonders gelenkbelastenden Tätigkeiten und beim Sport oder in einem Heilzeitraum nach einer Gelenkschädigung.

Das Anlegen solcher Kniebandagen erfolgt üblicherweise, indem die Kniebandage über den Fuß und Unterschenkel hinweg bis zum Knie gezogen wird. Das Ausziehen erfolgt in entsprechender Art und Weise. Als nachteilig an dieser Art des Anlegens und Ablegens der Kniebandage wird angesehen, dass ein An- und Ablegen der Bandage nicht gut über einen Schuh hinweg möglich ist. Bei Sportbandagen kommt hinzu, dass das Ausziehen nach dem Sport insbesondere durch eine erhöhte Haftneigung infolge von Schweißbildung auf der Haut erschwert ist.

Es sind Kniebandagen bekannt, die mittels dafür vorgesehener Spannelemente eine Anpassung der Kniebandagenspannung gestatten und die somit auch das Anlegen und Ablegen der Kniebandage erleichtern. Üblicherweise finden dabei Spannriemen, Haftbänder, Klettbänder und ähnliches Anwendung, die entweder vor oder nach dem Anlegen der Kniebandage anforderungsgerecht eingestellt werden.

Als nachteilig wird dabei angesehen, dass das Spannen und Entspannen der Kniebandagen mittels der Riemen zeitaufwendig ist und dass die ungleichmäßige Spannwirkung der Riemen Falten im Kniebandagenmaterial verursachen kann, die einerseits für den Träger der Kniebandage unbequem sind und andererseits die Wirkung der Kniebandage beeinträchtigen.

Aus dem Dokument US 5,653,244 A ist ein therapeutisches Kompressionskleidungsstück bekannt, das eine Mehrzahl an Paaren von körper- oder gliedmaßumkreisenden Bändern enthält, die ganzheitlich mit einem mittigen Umwickelbereich verbunden sind und die ausgehend von beiden Seiten des mittigen Umwickelbereichs in gegensätzliche Richtungen erstreckt sind, um das Körperteil zu umfassen. Ein longitudinal erstreckter Reißverschluss erstreckt sich zumindest über die longitudinale Länge des mittigen Bereichs des Kleidungsstücks, um das Entfernen und Wiederanbringen des Kleidungsstücks zu vereinfachen, ohne die Bänder zu lösen.

In dem Dokument US 2,280,025 A wird ein chirurgischer Strumpf beschrieben, der durch einen chirurgischen Strumpfkörper zum Anbringen an ein Bein gekennzeichnet ist, welcher eine offene Seite und einen Verschluss zum Verschließen dieser Seite aufweist, wobei der Verschluss beispielsweise einen Reißverschluss umfassen kann. Ein rückseitiger Streifen erstreckt sich entlang der Innenseite des Reißverschlusses und ist längs einer Seite des Reißverschlusses mittels einer Reihe von Stichen befestigt.

In dem Dokument DE 20207156 U1 wird ein medizinischer Kompressionsstrumpf aus Kunststoff oder textilem Gewebe beschrieben, der beispielsweise realisiert wird, indem ein medizinischer Kompressionsstrumpf auf der Rückseite der Länge nach von der Ferse zum Oberschenkel aufgeschnitten wird und mit einem Reißverschluss versehen wird.

Das Dokument GB 2077565 A beschreibt eine manschettenförmige Bandage oder ein manschettenförmiges Kleidungsstück für die Verwendung an einem Gliedmaß eines Tieres. Die Bandage umfasst einen Hüllkörper aus dehnbarem textilem Material, der entlang zweier longitudinaler Seitenkanten mit einem lösbaren Verschlussmittel ausgebildet ist, um die zwei Seitenkanten zusammenzuhalten, wenn der Überzug um das Gliedmaß positioniert ist. Das Verschlussmittel umfasst vorzugsweise einen Reißverschluss. An der Bandage ist auf der Innenseite longitudinal einseitig entlang des Reißverschlusses eine Lasche befestigt.

Der Erfindung liegt die Aufgabe zugrunde, eine Kniebandage der eingangs genannten Art zur Verfügung zu stellen, die ein einfaches Ausziehen bei dennoch hohem Tragekomfort ermöglicht.

Diese Aufgabe wird dadurch gelöst, dass eine Ausziehhilfe vorgesehen ist, die als durch einen Reißverschluss verschließbare Auftrennung gestaltet ist, und die sich über einen Teil der Länge oder über die vollständige Länge des Hüllkörpers erstreckt, wobei der Auftrennung auf einer dem Körperteil zugeordneten Innenseite des Hüllkörpers ein Schutzgebilde zugeordnet ist, das längs beider Seiten der Auftrennung mit dem Hüllkörper verbunden ist und das im angelegten Funktionszustand eine Zwischenlage zwischen einer Oberfläche des Kniebereiches und dem Reißverschluss bildet und das Schutzgebilde aus einem elastischen Material besteht, dessen Elastizitätsmodul in tangentialer Richtung geringer als ein Elastizitätsmodul des Hüllkörpers ist.

Der Hüllkörper hat im angelegten Funktionszustand eine im wesentlichen zylindermantelförmige oder konusmantelförmige Form, wobei die genaue Ausgestaltung und die Dimensionierung von der Zielgruppe und dem Einsatzzweck der Bandage bestimmt ist. Die Ausziehhilfe erstreckt sich in Längsrichtung des Hüllkörpers, wobei sie sich vorzugsweise zumindest an einer Stirnseite bis zum Ende des Hüllkörpers erstreckt.

Wenn die Auftrennung sich über die vollständige Länge der Kniebandage erstreckt, ist neben der herkömmlichen Art des Anlegens bei geschlossenem Reißverschluss auch ein Anlegen der Bandage dadurch möglich, dass die Bandage im geöffneten Zustand um das Knie gelegt wird und dann erst der Reißverschluss geschlossen wird. Insbesondere vereinfacht ist bei einer solchen Bandage mit einer Auftrennung über die gesamte Länge der Bandage das Ausziehen der Bandage. Durch Öffnen des Reißverschlusses wird die Bandage insgesamt an einer Seite geöffnet und löst sich dann wie von selbst vom Bein. Dadurch ist es nicht mehr erforderlich, die Bandage über die verschwitzte Haut hinweg Richtung Fuß zu ziehen.

Bei erfindungsgemäßen Kniebandagen, bei denen sich die Auftrennung nur über einen Teil der Länge der Bandage erstreckt, wird durch das Öffnen des Reißverschlusses eine tangentiale Aufweitung des Hüllkörpers erreicht. Diese Aufweitung führt zu einem geringen erforderlichen Kraftaufwand beim Ausziehen und ergänzend auch beim Anziehen der Kniebandage. Der Reißverschluss ermöglicht nach Anlegen der Kniebandage im aufgeweiteten Zustand ein schnelles und bequemes Herstellen der für die Kniebandagenwirkung erheblichen Tangentialspannung des Hüllkörpers. Bei einer solchen Kniebandage mit einem sich nicht über die Gesamtlänge erstreckenden Reißverschluss ist es insbesondere von Vorteil, dass das Einfädeln des Reißverschlussläufers entfällt, da der Reißverschlussläufer stets mit beiden Reißverschlussleisten verbunden ist. Ein solches Einfädeln ist besonders im angelegten Zustand aufgrund der erforderlichen Tangentialspannung im Hüllkörper schwierig.

Die im angelegten Funktionszustand bei geschlossenem Reißverschluss vorliegende Tangentialspannung des Hüllkörpers ist über die Länge der Auftrennung weitgehend homogen. Insbesondere für Menschen, die bezüglich ihrer Bewegungsfreiheit eingeschränkt sind, ist das Anlegen und Ablegen einer solchen Kniebandage gegenüber einem System mit Riemen, bei dem nacheinander mehrere Spannelemente zum Aufbau der Tangentialspannung gespannt werden müssen bzw. zum Lösen der Tangentialspannung geöffnet werden müssen, deutlich vereinfacht.

Erfindungsgemäß ist der Auftrennung auf einer dem Körperteil zugeordneten Innenseite des Hüllkörpers ein Schutzgebilde zugeordnet, das längs mindestens einer Seite der Auftrennung mit dem Hüllkörper verbunden ist und das im angelegten Funktionszustand eine Zwischenlage zwischen einer Oberfläche des Kniebereiches und dem Reißverschluss bildet.

Das Schutzgebilde ist dabei vorzugsweise aus einem flexiblen, insbesondere einem textilen, Material gefertigt. Es erfüllt den Zweck, die Hautoberfläche und den Reißverschluss voneinander zu trennen. Dies erhöht den Tragekomfort, da ansonsten aufgrund der Relativbewegung zwischen Reißverschluss und Hautoberfläche eine Reizung oder Verletzung der Haut möglich ist. Darüber hinaus wird bei metallischen Reißverschlüssen das subjektiv unangenehme Gefühl von kaltem Metall auf der Haut vermieden. Ein weiterer Vorteil ist, dass durch das Schutzgebilde beim Öffnen und Schließen des Reißverschlusses im angelegten Funktionszustand vermieden wird, dass Haut oder Haare in den Reißverschluss gelangen.

Wenn das Schutzgebilde lediglich mit einer der beiden Seiten der Auftrennung verbunden ist, ist es so dimensioniert, dass es sich im angelegten Zustand tangential innenliegend über den Reißverschluss hinweg erstreckt, so dass ein direkter Kontakt zwischen Haut und Reißverschluss vermieden wird. Bei einem solchen mit nur einer Seite der Auftrennung verbundenen Schutzgebilde ist das Material des Schutzgebildes vorzugsweise von geringer Flexibilität, so dass es nicht beim Anlegen oder im angelegten Zustand Falten wirft und dadurch keinen sichere Trennung zwischen Haut und Reißverschluss mehr darstellt. Vorzugsweise ist das Schutzgebilde beidseitig der Längskanten der Auftrennung mit dem Hüllkörper verbunden. Wenn das Schutzgebilde beidseitig mit den benachbarten Längsseiten an der Auftrennung verbunden ist, ist es so zu dimensionieren und/oder bezüglich des Materials auszubilden, dass es einer Aufweitung des Hüllkörpers nach Öffnen des Reißverschlusses nicht oder kaum entgegensteht. Ein solches beidseitig der Auftrennung mit dem Hüllkörper verbundenes Schutzgebilde erfüllt auch den Zweck, dass es beim Anlegen der Kniebandage eine Führung für das Körperteil darstellt, so dass der Tragende nicht versehentlich, beispielsweise mit einem Zeh, in den Reißverschluss gerät.

Erfindungsgemäß besteht das Schutzgebilde aus einem elastischen Material, dessen Elastizitätsmodul in tangentialer Richtung geringer als der Elastizitätsmodul des Hüllkörpers ist. Dies ist vor allem bei einem beidseitig der Auftrennung mit dem Hüllkörper verbundenen Schutzgebilde zweckmäßig. Das elastische Material des Schutzgebildes ermöglicht es, dass das Schutzgebilde einerseits im ungespannten Zustand des Hüllkörpers eine zum Anlegen und Ausziehen der Kniebandage ideale Hüllkörperaufweitung erlaubt, während andererseits vermieden wird, dass in dem mit dem Reißverschluss gespannten Zustand der Kniebandage das Schutzgebilde Falten oder Wülste verursacht, die für den Tragenden auf der Haut unangenehm sind. Durch den gegenüber dem Hüllkörper geringeren E-Modul ist gewährleistet, dass bei geöffnetem Reißverschluss, beispielsweise zum Ausziehen und Anlegen der Kniebandage, ein Aufweiten des Hüllkörpers problemlos möglich ist, da die dafür erforderliche Dehnung ohne hohen Kraftaufwand durch das Schutzgebilde ermöglicht wird. Eine Dehnung des Hüllkörpermaterials selbst ist zur Aufweitung dagegen nicht erforderlich.

In einer Weiterbildung der Erfindung ist an mindestens einem Endbereich des Reißverschlusses ein Schutzabschnitt, insbesondere ein Schutzpolster, vorgesehen, welcher im angelegten Funktionszustand der Kniebandage zwischen dem Endbereich des Reißverschlusses und der Oberfläche des Kniebereiches angeordnet ist.

Der Schutzabschnitt besteht dabei vorzugsweise aus einem textilen Material. Er schirmt die Hautoberfläche von den beim Hautkontakt als unangenehm empfundenen Endbereichen des Reißverschlusses ab. Der Schutzabschnitt kann an einer Seite der Auftrennung am Hüllkörper befestigt oder auch einstückig mit dem Hüllkörper ausgebildet sein. Alternativ kann der Schutzabschnitt auch mit dem Schutzgebilde verbunden sein.

In einer darauf aufbauenden Weiterbildung der Erfindung ist ein Sicherungsabschnitt des Schutzabschnittes auf die Außenseite des Hüllkörpers der Kniebandage umklappbar, und auf der Außenseite des Hüllkörpers sind Fixiermittel zum lösbaren Fixieren des Sicherungsabschnitts vorgesehen. Der Sicherungsabschnitt ist vorzugsweise aus einem textilen Material gefertigt. Er kann einstückig mit einem innenliegenden Teilabschnitt des Schutzabschnittes verbunden sein. Die Fixiermittel können beispielsweise als Druckknöpfe oder als Klettverschluss ausgebildet sein. Durch den Sicherungsabschnitt wird erreicht, dass es nicht zu einer Berührung des Endbereichs des Reißverschlusses mit der Haut infolge der Bewegung des Knies und einem damit verbundenen Verrutschen des Schutzabschnittes kommt.

In einer Weiterbildung der Erfindung ist der Schutzabschnitt mit dem Schutzgebilde verbunden, insbesondere vernäht, oder einteilig mit dem Schutzgebilde ausgebildet.

Eine solche Kombination von Schutzabschnitt und Schutzgebilde ist leicht und kostengünstig zu fertigen. Eine solche Ausführung ist insbesondere bei einem Schutzgebilde vorteilhaft, das nur an einer Seite der Auftrennung mit dem Hüllkörper verbunden ist. Die Fixiermittel zur Befestigung des Schutzabschnittes dienen dort dann gleichzeitig dazu, das Schutzgebilde zu straffen und ein Verrutschen zu verhindern.

In einer Weiterbildung der Erfindung weist mindestens eine Längsseite der Auftrennung einen Stützstrang auf, der eine geringere Flexibilität als das Hüllkörpermaterial aufweist.

Dieser Stützstrang kann beispielsweise durch zwei oder mehr miteinander vernähten Streifenabschnitte des Hüllkörpermaterials gebildet werden. Das Vorsehen eines solchen erfindungsgemäßen Stützstranges ist sowohl beim Anlegen der Kniebandage bei geöffnetem Reißverschluss als auch im Funktionszustand zweckmäßig. Beim Anlegen der Kniebandage wird durch den Stützstrang verhindert, dass sich das Hüllkörpermaterial umschlägt und ein Schließen des Reißverschlusses erschwert wird. Im gespannten Funktionszustand bei geschlossenem Reißverschluss stellt der Stützstrang eine zusätzliche Stabilisierung des zu stützenden Kniegelenkes, insbesondere in Querrichtung, dar und beugt so einer orthopädisch nachteiligen Stellung des Gelenks vor.

In einer Weiterbildung der Erfindung umfasst der Stützstrang eine Stützstrebe, die vorzugsweise aus Kunststoff gefertigt ist und in dem elastischen Material des Hüllkörpers zumindest abschnittsweise eingebettet ist.

Eine solche Stützstrebe stabilisiert das Knie besonders gut und schützt so das Kniegelenk. Die Stützstrebe kann so ausgebildet sein, dass sie quer zu einer Abknickrichtung des Kniegelenkes eine höhere Steifigkeit als in Abknickrichtung aufweist.

In einer Weiterbildung der Erfindung ist die Auftrennung im angelegten Funktionszustand an einer Seite des Kniebereiches angeordnet.

Die Anordnung des Anzieh- und/oder Ausziehhilfe an der Seite des Kniebereiches stellt die geringstmögliche Einschränkung bezüglich des Tragekomforts für den Tragenden dar. Während an der Vorder- und an der Rückseite sich in Abhängigkeit der Beugestellung des Knies der Abstand zwischen den beiden Stirnseiten der Kniebandage permanent verändert, ist der Abstand der Stirnseiten an den Seiten des Beines weitgehend konstant. Die Anzieh- und/oder Ausziehhilfe kann an der Innenseite oder der Außenseite vorgesehen sein.

Weitere Vorteile und Merkmale der Erfindung ergeben sich aus den Ansprüchen sowie aus der nachfolgenden Beschreibung zweier vorteilhafter Ausführungsformen, die in den Zeichnungen dargestellt sind.
- Fig. 1: zeigt eine Ausführungsform einer erfindungsgemäßen Kniebandage in einer Seitendarstellung im entspannten Zustand mit aufgeweitetem Hüllkörper und geöffnetem Reißverschluss, der sich über einen Teil der Länge der Kniebandage erstreckt,
- Fig. 2: zeigt die in Figur 1 dargestellte Ausführungsform der Kniebandage im gespannten Zustand mit geschlossenem Reißverschluss,
- Fig. 3.: zeigt eine Ausführungsform einer beispielhaften Kniebandage in einer Seitendarstellung im entspannten Zustand mit aufgeweitetem Hüllkörper und geöffnetem Reißverschluss, der sich über die Gesamtlänge der Kniebandage erstreckt und
- Fig. 4: zeigt die in Figur 3 dargestellte Ausführungsform der Kniebandage im gespannten Zustand mit geschlossenem Reißverschluss.

Die Bezugszeichen beider Ausführungsformen sind bei gleichen oder annähernd gleichen Komponenten identisch.

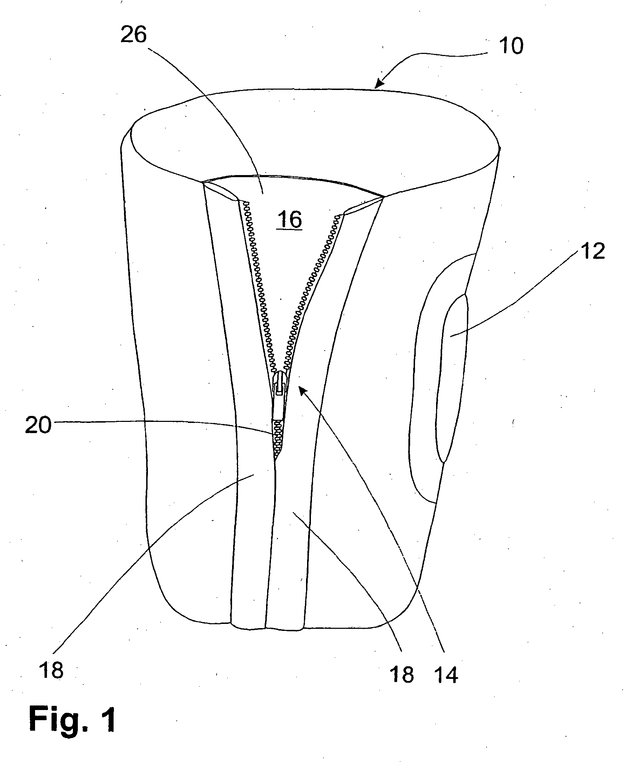

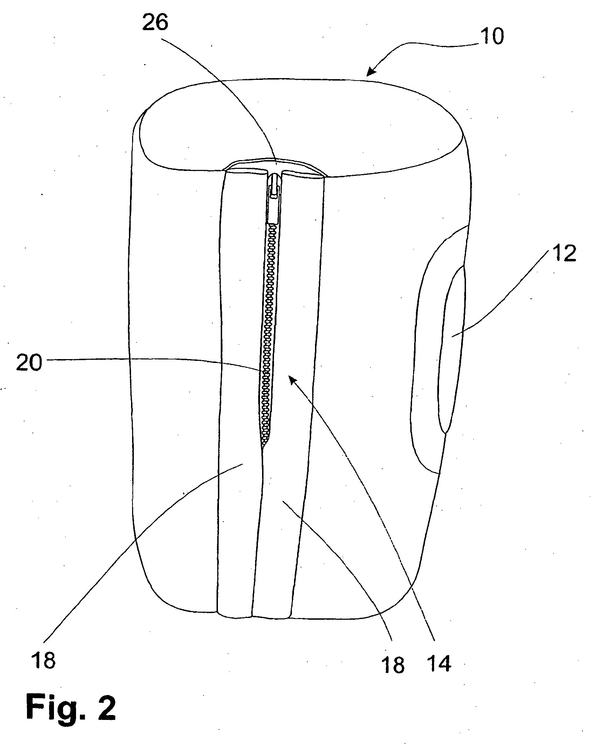

Die Figuren 1 und 2 zeigen eine Ausführungsform einer erfindungsgemäßen Kniebandage. Die Kniebandage weist einen zylindermantelförmigen Hüllkörper 10 auf, der auf einer Vorderseite mit einem Knieschutz 12 ausgestattet ist. An einer Seite der Kniebandage ist ein Anzieh-/Ausziehhilfe 14 vorgesehen. Diese erstreckt sich von der oberen Stirnseite des Hüllkörpers 10 in etwa über zwei Drittel der Länge des Hüllkörpers 10 nach unten. Diese Anzieh-/Ausziehhilfe 14 weist eine Auftrennung 16 auf. Parallel und benachbart zur Auftrennung 16 verlaufend sind zwei sich in Längsrichtung erstreckende Stutzkanten 18 vorgesehen, deren Flexibilität geringer ist als die Flexibilität des Hüllkörpers 10. Die beiden Stützkanten 18 sind durch einen Reißverschluss 20 verbindbar, der aus zwei Seitenleisten sowie aus einem Schieber besteht. Die Seitenleisten sind an den Stutzkanten 18 festgenäht. Auf der Innenseite der Anzieh-/Ausziehhilfe 14 ist ein textiles Schutzgebilde 26 vorgesehen, welches aus einem stark dehnbaren Material besteht. Das Schutzgebilde 26 ist beidseitig der Auftrennung 16 mit den Stützkanten 18 vernäht und erstreckt sich in Längsrichtung des Hüllkörpers 10 über die gesamte Länge der Auftrennung 16.

Die Figur 1 zeigt den entspannten Zustand. In diesem Zustand ist der Reißverschluss 20 weitgehend geöffnet, so dass der Umfang der Kniebandage an der oberen Stirnseite gegenüber einem gespannten Zustand mit geschlossenem Reißverschluss 20 vergrößert ist. Zum Schutz des Beines ist in diesem entspannten Zustand bei geöffnetem Reißverschluss 20 das Schutzgebilde 26 vorgesehen, welches in Folge der Aufweitung der Kniebandage stark gedehnt ist. In dem dargestellten Zustand ist das Anlegen der Kniebandage bequem möglich. Durch den vergrößerten Umfang an der oberen Stirnseite kann das Bein sehr einfach in die Kniebandage eingeführt werden, ohne dass schon im Bereich der Ferse oder des Unterschenkels eine hohe Reibkraft zwischen Kniebandage und Körperteil das Anlegen erschwert. Das Schutzgebilde 26 verhindert, dass beim Anlegen Haut oder Haare in den Reißverschluss 20 gelangen. Wenn die Kniebandage in der gewünschten Stellung, im vorliegenden Fall also im Bereich des Knies, auf richtiger Höhe sitzt, kann durch Schließen des Reißverschlusses 20 die aus orthopädischen Gesichtspunkten gewünschte Spannung hergestellt werden. Auch beim Schließen des Reißverschlusses 20 verhindert das Schutzgebilde 26, dass Haut oder Haare in Mitleidenschaft gezogen werden. In diesem Zustand bei geöffnetem Reißverschluss 20 ist darüber hinaus auch das Ausziehen der Kniebandage wesentlich erleichtert, da aufgrund der teilweisen Entspannung der Kniebandage eine geringere Kraft erforderlich ist, um die Kniebandage über den Unterschenkel und den Fuß zu ziehen.

Figur 2 zeigt die Kniebandage in angelegtem Funktionszustand. Es ist zu erkennen, dass der Reißverschluss 20 geschlossen ist, so dass die Kniebandage die weitgehend zylindermantelförmige Form aufweist. Das Schutzgebilde 26 ist in diesem Zustand nicht mehr gedehnt. Durch die Elastizität des Schutzgebildes 26 ist gewährleistet, dass das Schutzgebilde 26 in dem in Figur 2 dargestellten Zustand der Kniebandage bei geschlossenem Reißverschluss 20 keine Falten wirft. Stattdessen liegt es glatt am Bein an und trägt damit zum hohen Tragekomfort der Kniebandage bei. Durch den geschlossenen Reißverschluss 20 kann eine tangentiale Dehnung der Kniebandage nur noch über eine Dehnung des Hüllkörpers 10 erfolgen. Da hierfür jedoch verglichen mit dem Stützgebilde 26 eine wesentlich höhere Kraft erforderlich ist, gewährleistet die Kniebandage die gewünschte permanente Stütz- und Kompressionswirkung auf das Knie. Die Stützkanten 18 verlaufen im gespannten Zustand der Kniebandage weitgehend parallel und stabilisieren das Kniegelenk insbesondere in Querrichtung.

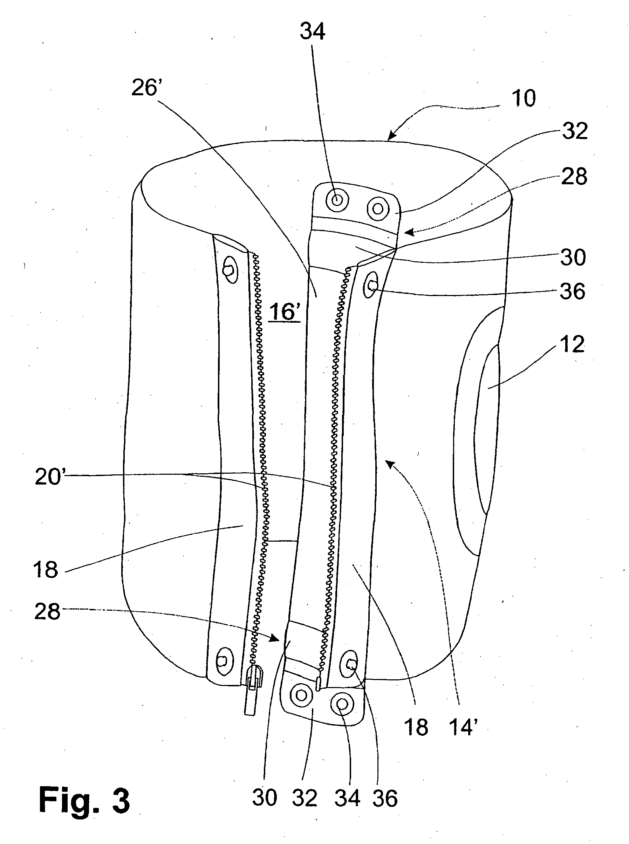

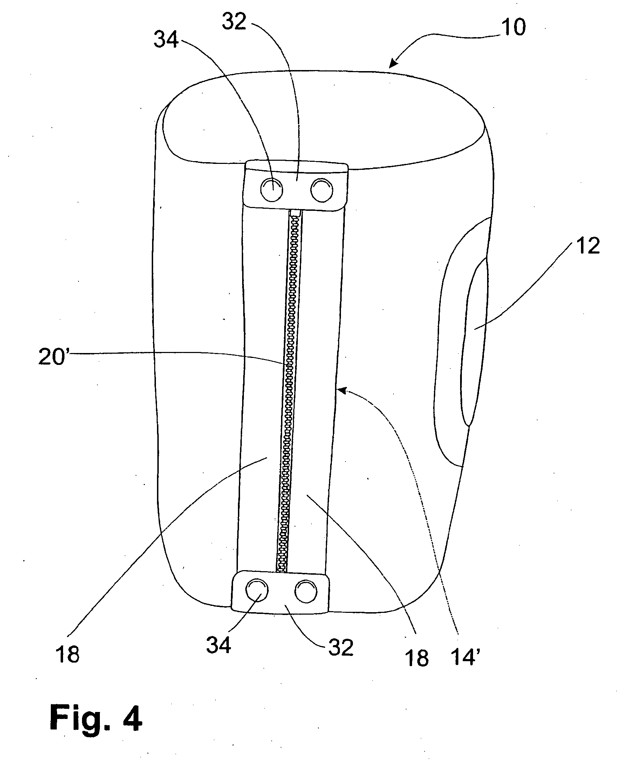

Die in den Figuren 3 und 4 dargestellte Ausführungsform der beispielhaften Kniebandage unterscheidet sich zu der Ausführungsform der erfindungsgemäßen Kniebandage in verschiedenen Punkten. Die Anzieh-/Ausziehhilfe 14' mit der Auftrennung 16' und dem Reißverschluss 20' erstreckt sich bei dieser zweiten Ausführungsform über die gesamte Länge der Kniebandage. Das Schutzgebilde 26' ist nur an einer Seite mit dem Hüllkörper 10 der Kniebandage verbunden und weist eine geringere Flexibilität als das Schutzgebilde 26 der ersten Ausführungsform auf. Am unteren und am oberen Ende ist das Schutzgebilde 26' einstückig mit Schutzpolstern 28 verbunden. Diese weisen einen Teilabschnitt 30 auf, der so angeordnet ist, dass er im angelegten Funktionszustand die Endbereiche des Reißverschlusses 20' von der Hauptoberfläche trennt. Darüber hinaus weisen die Schutzpolster 28 jeweils einen Sicherungsabschnitt 32 auf, der im angelegten Funktionszustand auf die Außenseite des Hüllkörpers geklappt wird und dort mit Fixiermitteln befestigt wird. Die Fixiermittel sind bei der dargestellten Ausführungsform als jeweils zwei Druckknöpfe je Schutzpolster 28 ausgeführt, wobei die Sicherungsabschnitte 32 der Schutzpolster 28 jeweils zwei Druckknopf-Buchsenabschnitte 34 aufweisen, während auf den Stützkanten 18 die dazu korrespondierenden Druckknopf-Steckerabschnitte 36 vorgesehen sind.

Figur 3 zeigt die Ausführungsform der beispielhaften Kniebandage bei geöffnetem Reißverschluss 20'. In diesem Zustand sind die beiden Stützkanten 18 vollständig voneinander gelöst, so dass ein Anlegen und Ablagen der Bandage möglich ist, ohne sie über Unterschenkel 30 und Fuß ziehen zu müssen. Die Druckknöpfe 34, 36 sind geöffnet, so dass die Endbereiche des Reißverschlusses 20' freiliegen.

Figur 4 zeigt die Kniebandage im angelegten Funktionszustand. Der Reißverschluss 20 ist vollständig geschlossen. Die Sicherungsabschnitte 32 der Schutzpolster 28 sind am oberen und unteren Ende der Kniebandage über die Stirnenden des Hüllkörpers 10 geklappt und auf der Außenseite der Bandage mittels der Druckknöpfe 34, 36 gesichert, indem die Druckknopf-Buchsenabschnitte 34 auf den Druckknopf-Steckerabschnitten 36 arretiert sind. Dadurch ist gewährleistet, dass die Schutzabschnitte 30 der Schutzpolster 28 auch bei starker Bewegung des Knies nicht verrutschen und dadurch einen direkten Kontakt der Endbereiche des Reißverschlusses 20' mit der Haut zulassen.

## Patentansprüche

1. Medizinische Kniebandage mit einem Hüllkörper (10) aus zumindest abschnittsweise elastischem Material, der an gegenüberliegenden Stirnseiten offen ist und in angelegtem Funktionszustand einen geschlossenen und um einen menschlichen Kniebereich tangential elastisch gespannten Hüllmantel bildet,
wobei
- eine Ausziehhilfe (14; 14') vorgesehen ist, die als durch einen Reißverschluss (20; 20') verschließbare Auftrennung (16; 16') gestaltet ist, und die sich über einen Teil der Länge oder über die vollständige Länge des Hüllkörpers (10) erstreckt,
**dadurch gekennzeichnet, dass**
- der Auftrennung (16; 16') auf einer dem Körperteil zugeordneten Innenseite des Hüllkörpers (10) ein Schutzgebilde (26; 26') zugeordnet ist, das längs beider Seiten der Auftrennung (16; 16') mit dem Hüllkörper (10) verbunden ist und das im angelegten Funktionszustand eine Zwischenlage zwischen einer Oberfläche des Kniebereiches (16; 16') und dem Reißverschluss (20; 20') bildet und
- das Schutzgebilde (26) aus einem elastischen Material besteht, dessen Elastizitätsmodul in tangentialer Richtung geringer als ein Elastizitätsmodul des Hüllkörpers (10) ist.

2. Kniebandage nach Anspruch 1,
**dadurch gekennzeichnet, dass**
an mindestens einem Endbereich des Reißverschlusses (20') ein Schutzabschnitt, insbesondere ein Schutzpolster (28), vorgesehen ist, welcher im angelegten Funktionszustand der Kniebandage zwischen dem Endbereich des Reißverschlusses (20') und der Oberfläche des Kniebereiches angeordnet ist.

3. Kniebandage nach Anspruch 2,
**dadurch gekennzeichnet, dass**
ein Sicherungsabschnitt (32) des Schutzabschnittes (28) auf die Außenseite des Hüllkörpers (10) der Kniebandage umklappbar ist und dass auf der Außenseite des Hüllkörpers (10) Fixiermittel (36) zum lösbaren Fixieren des Sicherungsabschnitts (28) vorgesehen sind.

4. Kniebandage nach Anspruch 2 oder 3,
**dadurch gekennzeichnet, dass**
der Schutzabschnitt (28) mit dem Schutzgebilde (26') verbunden, insbesondere vernäht, ist oder einteilig mit dem Schutzgebilde (26') ausgebildet ist.

5. Kniebandage nach Anspruch 1,
**dadurch gekennzeichnet, dass**
mindestens eine Längsseite der Auftrennung einen Stützstrang (18) aufweist, der eine geringere Flexibilität als das Material des Hüllkörpers (10) aufweist.

6. Kniebandage nach Anspruch 5,
**dadurch gekennzeichnet, dass**
der Stützstrang (18) eine Stützstrebe umfasst, die vorzugsweise aus Kunststoff gefertigt ist und in dem elastischen Material des Hüllkörpers (10) zumindest abschnittsweise eingebettet ist.

7. Kniebandage nach einem der vorstehenden Ansprüche,
**dadurch gekennzeichnet, dass** die Auftrennung (16, 16') im angelegten Funktionszustand an einer Seite des Kniebereiches angeordnet ist.

## Claims

1. Medical knee bandage with a sheathing body (10) made of material that is elastic at least in sections, which sheathing body is open on opposing face sides, and, in an applied functional state, forms a closed sheathing jacket that is tangentially elastically strained about a human knee area, wherein
- a removal aid (14, 14') is provided, which is designed as a separation (16, 16') that is closeable by a zipper (20, 20'), and which extends over a part of the length or over the entire length of the sheathing body (10), **characterized in that**
- a protective structure (26, 26') is allocated to the separation (16, 16') on an inside of the sheathing body (10) that is allocated to the body part, the protective structure (26, 26') being joint with the sheathing body (10) along both sides of the separation (16, 16'), and forming an intermediate layer between a surface of the knee area (16, 16') and the zipper (20, 20') in the applied functional state, and
- the protective structure (26) consisting of an elastic material, whose modulus of elasticity in tangential direction is lower than a modulus of elasticity of the sheathing body (10).

2. The knee bandage according to claim 1,
**characterized in that** a protective section, particularly a protective cushion (28), is provided at at least one end area of the zipper (20'), which protective section is arranged between the end area of the zipper (20') and the surface of the knee area in the applied functional state of the knee bandage.

3. Knee bandage according to claim 2,
**characterized in that** a securing section (32) of the protective section (28) is foldable onto the outside of the sheathing body (10) of the knee bandage, and that fixation means (36) for detachably fixing the securing section (28) are provided on the outside of the sheathing body (10).

4. Knee bandage according to claim 2 or 3,
**characterized in that** the protective section (28) is joint, particularly sewed with the protective structure (26'), or formed in one piece with the protective structure (26').

5. Knee bandage according to claim 1,
**characterized in that** at least one longitudinal side of the separation has a supporting strand (18), which has a lower flexibility than the material of the sheathing body (10).

6. Knee bandage according to claim 5,
**characterized in that** the supporting strand (18) comprises a supporting strut, which is preferably made of plastic, and which is at least in sections embedded in the elastic material of the sheathing body (10).

7. Knee bandage according to one of the preceding claims,
**characterized in that** the separation (16, 16') is arranged on one side of the knee area in the applied functional state.

## Revendications

1. Genouillère médicale comprenant un corps enveloppant (10) en matière au moins par endroits élastique, lequel corps est ouvert sur des côtés frontaux opposées et forme, en position de fonctionnement, un manteau enveloppant fermé et tendu tangentiellement et élastiquement autour d'une zone de genou humain,
dans le cadre de laquelle
- il est prévu une aide au retrait (14 ; 14') qui est réalisée sous la forme d'une ouverture (16 ; 16') pouvant être fermée à l'aide d'une fermeture à glissière (20 ; 20') et s'étend sur une partie de la longueur ou sur toute la longueur dudit corps enveloppant (10),
**caractérisée en ce que**
- une structure de protection (26 ; 26') est associée à ladite ouverture (16 ; 16') sur une face intérieure du corps enveloppant (10) attribuée à la partie du corps, laquelle structure est reliée au corps enveloppant (10) le long des deux côtés de l'ouverture (16 ; 16') et forme, en position de fonctionnement, une couche intermédiaire entre une surface de la zone de genou et la fermeture à glissière (20 ; 20') et
- ladite structure de protection (26) est constituée d'une matière élastique dont le module d'élasticité est, dans la direction tangentielle, inférieure à un module d'élasticité du corps enveloppant (10).

2. Genouillère médicale selon la revendication 1,
**caractérisée en ce que**
il est prévu au moins à une extrémité de la fermeture à glissière (20') un segment de protection, plus particulièrement un rembourrage de protection (28), lequel est disposé entre l'extrémité de la fermeture à glissière (20') et la surface de la zone de genou lorsque la genouillère se trouve en position de fonctionnement.

3. Genouillère médicale selon la revendication 2,
**caractérisée en ce que**
un segment de fixation (32) dudit rembourrage de protection (28) est repliable sur la face extérieure du corps enveloppant (10) de la genouillère et que des moyens de fixation (36) sont prévus sur la face extérieure du corps enveloppant (10) pour la fixation amovible du segment de fixation (28).

4. Genouillère médicale selon la revendication 2 ou 3,
**caractérisée en ce que**
ledit segment de protection (28) est relié à la structure de protection (26'), plus particulièrement par le biais d'une couture, ou réalisé d'une seule pièce avec la structure de protection (26').

5. Genouillère médicale selon la revendication 1,
**caractérisée en ce que**
au moins un côté longitudinal de l'ouverture présente une bande de soutien (18) qui présente une souplesse plus faible que la matière du corps enveloppant (10).

6. Genouillère médicale selon la revendication 5,
**caractérisée en ce que**
ladite bande de soutien (18) comporte un renfort qui est réalisé préférentiellement à partir d'une matière synthétique et incorporé au moins par endroits dans la matière élastique du corps enveloppant (10).

7. Genouillère médicale selon l'une des revendications précédentes,
**caractérisée en ce qu'**en position de fonctionnement, l'ouverture (16, 16') est disposée sur un côté de la zone de genou.
